# EUROPEAN PATENT APPLICATION

(11) **EP 3 111 967 A1**
(43) Date of publication of application: **04.01.2017**
(21) Application number: 14883944.2
(22) Date of filing: 19.08.2014
(51) Int. Cl.: A61L 27/42, A61L 27/56, A61L 27/02

(54) **HIGH STRENGTH SYNTHETIC BONE FOR BONE REPLACEMENT FOR INCREASING COMPRESSIVE STRENGTH AND FACILITATING BLOOD CIRCULATION, AND MANUFACTURING METHOD THEREFOR**

(30) Priority: 26.02.2014 KR 20140022572
(71) Applicant: Ossein Co. Ltd., Wonju-si, Gangwon-do 220-712 (KR)
(72) Inventor: PARK, Kyeong Jun, Goyang-si Gyeonggi-do 410-360 (KR); PARK, Seok Bong, Yeongwol-gun Gangwon-do 230-835 (KR); SHIN, Jae Oh, Seoul 151-015 (KR)
(74) Representative: Grünecker Patent- und Rechtsanwälte PartG mbB
(86) International application number: PCT/KR2014/007683
(87) International publication number: WO 2015/129972

(57) **Abstract**

The present invention relates to a high strength synthetic bone for bone replacement for increasing compressive strength and facilitating blood circulation, and a manufacturing method therefor, and provides the high strength synthetic bone for bone replacement in which calcium sulfate hemihydrate (CSH) and NaCl, in a particle state, penetrate into the pores of a porous inorganic material such as β-tricalcium phosphate (β-TCP) and a wet treatment is performed on the same such that the CSH penetrated into the pores is combined with moisture so as to form a hydrated crystal of calcium sulfate dihydrate (CSD) to expand the volume thereof in the pores, thereby preventing the escape of a filler by physical force.

## Description

### [Technical Field]

The present invention relates to a novel synthetic bone for bone replacement made of an inorganic material and a manufacturing method therefor, and more particularly, to a synthetic bone for bone replacement capable of increasing strength and facilitating blood circulation by penetrating plaster and NaCl, wherein a volume of the plaster expands as calcium sulfate hemihydrate (CSH) in a particle state is filled into pores of a porous inorganic material such as β-tricalcium phosphate (β-TCP) and then combined with moisture to be converted into calcium sulfate dihydrate (CSD), and a manufacturing method therefor.

### [Background Art]

Bones have a mechanical function of supporting the human body and helping the human body work well, and also serve as a reservoir for calcium while adjusting a concentration of calcium ions in the body and has an important physiological function of producing red and white blood cells required in the human body. Bones may be damaged due to aging and other physiological reasons, or may be damaged due to various accidents.

Bone grafting includes a method of grafting a patient's own tissue (autologous bone grafting), a method of grafting a bone derived from other people (allogeneic bone grafting) or an animal (xenogenic bone grafting), etc. However, when an immunological rejection reaction occurs due to the grafting of a tissue derived from other people, or a material that may be used in a patient's body is not sufficient due to a large affected site, artificial bone graft materials (bone substitutes) have been used.

Most currently used synthetic bones are based on calcium sulfate and calcium phosphate, but have limitations in having an effect in autologous bone grafts.

Calcium phosphate is an inorganic material that has received much attention as a bone substitute due to its similarity to a composition of natural bone and superior osteoconductivity. Brown *et al.* conducted research on porous hydroxyapatite (hereinafter referred to as HA) having an absorbing property among the inorganic materials, and Wolfe reported that β-tricalcium phosphate (hereinafter referred to as β-TCP) is slowly decomposed and substituted by new bone since β-TCP has a structure similar to an inorganic component of natural bone. Chow *et al.* reported on the osteoconductivity of β-TCP. In addition, Posset *et al.* reported research on tetracalcium phosphate, and Frankenburg et al. reported research on calcium phosphate cement, etc. Also, there is research on preparation into the form of bone cement in which an inorganic material is not used alone but various inorganic materials are mixed. It is judged to be a great idea in that this material is not in the form of powder but has viscosity, and thus inhibits initial fluidity and maintains its shape to some degree, but it is not sufficient to expect satisfactory results. Most of these materials have restrictions on sites in which they can be used since they are supplied in the form of powder, or have poor strength and a difficulty in maintaining their shapes during contouring although they are not supplied in the form of powder.

Since these biological ceramic materials for bone regeneration are not osteoinductive but osteoconductive materials, they may be used as porous materials which have connected pores having a suitable size so that bone tissue can enter to grow in the materials, and have a desired property such that they have a biodegradation rate similar to a growth rate of new bone.

### [Disclosure]

### [Technical Problem]

Based on this research, the present inventors have endeavored to conduct much research in order to overcome problems such as low strength and low replacement of new bones, and found that a high-strength inorganic material for bone replacement, in which NaCl and plaster whose volume expands as calcium sulfate hemihydrate (CSH) in a particle state is filled into pores of a porous inorganic material such as β-TCP and then combined with moisture to be converted into calcium sulfate dihydrate (CSD) are penetrated in a particle state to fill the pores, a density of the material is enhanced to increase strength, and the phase equilibrium is induced with NaCl, etc. to improve new bone conduction capability, may be manufactured so as to synthesize a novel inorganic material for bone replacement which has rapid new bone conduction capability and high strength while maintaining a bioactive property. Therefore, the present invention has been completed based on these facts.

Therefore, it is an aspect of the present invention to provide a high-strength synthetic bone for bone replacement having enhanced strength and new bone conduction capability by penetrating plaster and NaCl into pores of a porous inorganic material.

### [Technical Solution]

To solve the above problems, one aspect of the present invention provides a synthetic bone for bone replacement including a porous inorganic material; plaster filled into pores of the porous inorganic material; and NaCl filled into the pores of the porous inorganic material.

In the present invention, the porous inorganic material may be at least one or a mixture of two or more selected from the group consisting of β-tricalcium phosphate, α-tricalcium phosphate, dicalcium phosphate dibasic, tetracalcium phosphate, hydroxyapatite, calcium phosphate cement, calcium carbonate, calcium sulfate, a bioactive glass ceramic, and silica.

In the present invention, a volume of the plaster may expand as calcium sulfate hemihydrate in a particle state is filled into the pores of the porous inorganic material and then combined with moisture to be converted into calcium sulfate dihydrate.

The synthetic bone for bone replacement according to the present invention may further include a polysaccharide filled into the pores of the porous inorganic material.

In the present invention, each of the plaster and NaCl is preferably filled in a particle state having a diameter of 100 µm or less.

In the present invention, a mixing ratio of the NaCl and plaster is preferably in a range of 1:4 to 1:99 (based on weight).

Another aspect of the present invention provides a method for manufacturing a synthetic bone for bone replacement, which includes mixing plaster and NaCl with a porous inorganic material to fill the plaster and NaCl into pores of the porous inorganic material; wet-treating the porous inorganic material filled with the plaster and NaCl; and drying the wet-treated porous inorganic material.

### [Advantageous Effects]

According to the present invention, the high-strength synthetic bone for bone replacement can increase ease in molding a pre-surgical material during surgery for implantation of a bone substitute to fill a bone defect and may maintain a shape of a molded product in an original state in the body for a predetermined period of time after the surgery since the high-strength synthetic bone for bone replacement has an increased compressive strength. At the same time, the high-strength synthetic bone for bone replacement is expected to be widely used as a superior material capable of replacing conventional synthetic bones for bone replacement since a change in concentration of a body fluid in pores of an inorganic material with the dissolution of NaCl as a filler induces a phase equilibrium reaction similar to osmosis to improve blood circulation in the pores.

### [Description of Drawings]

FIGS. 1 to 4 are microscope images showing surface morphologies of high-strength synthetic bones for bone replacement according to the present invention: FIG. 1A is an image of cylindrical β-TCP at the beginning, FIG. 2B is an image of cylindrical β-TCP after plaster is mixed with NaCl, FIG. 3C is an image of cylindrical β-TCP after 1 week of impregnation into a simulated body fluid (SBF), and FIG. 4D is an image of cylindrical β-TCP after 2 weeks of impregnation into SBF.
FIG. 5 is a graph illustrating a change in compressive strength according to conditions for deposition of SBF of the high-strength synthetic bone for bone replacement according to the present invention.
FIG. 6 is a graph illustrating a change in compressive strength of the high-strength synthetic bone for bone replacement according to a change in content of NaCl when fillers (CSH and NaCl) are penetrated.

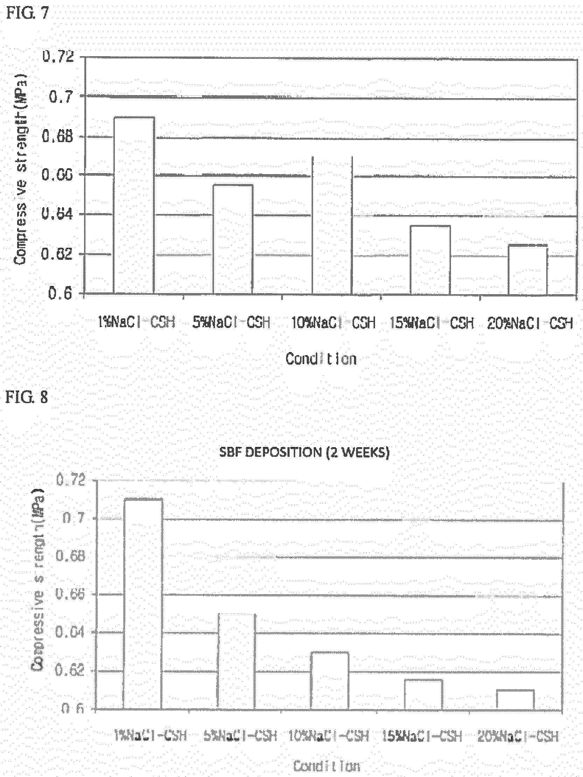

FIG. 7 is a graph illustrating a change in compressive strength of the high-strength synthetic bone for bone replacement according to a change in content of NaCl when deposited in SBF for 1 week.
FIG. 8 is a graph illustrating a change in compressive strength of the high-strength synthetic bone for bone replacement according to a change in content of NaCl when deposited in SBF in for 2 weeks.

### [Best Mode]

Hereinafter, the present invention will be described in detail.

The present invention is directed to a high-strength synthetic bone for bone replacement and a manufacturing method therefor.

The high-strength synthetic bone for bone replacement according to the present invention may include a porous inorganic material; plaster filled into pores of the porous inorganic material; and NaCl filled into the pores of the porous inorganic material.

The high-strength synthetic bone for bone replacement according to the present invention is characterized by filling a portion of the pores of the porous inorganic material for bone replacement with an additive (CSH, NaCl, etc.) which is easily dissolved in the body.

In the present invention, the inorganic material is preferably a porous and osteoconductive inorganic material. The inorganic material is preferably used as a porous material which has connected pores having a suitable size so that bone tissue can enter to grow in the materials. The pores formed inside the inorganic material are preferably at least partially connected to each other. The size of the pores is preferably less than or equal to 500 µm, and 100 µm. The porous inorganic material may be manufactured in the form of bone, and may also be manufactured in various shapes such as a cylindrical shapes, etc.

For the inorganic material for bone replacement according to the present invention, inorganic materials that may be absorbed into the body or stay in the body like inorganic components of a bone, and may conduct bone formation may be used as the inorganic material for bone replacement. For example, hydroxyapatite (HA: Ca₁₀(PO₄)₆(OH)₂), calcium phosphate cement, calcium carbonate, calcium sulfate, tricalcium phosphate (TCP), bioplast hard tissue replacement (HTR), a bioactive glass ceramic, silica, and the like may be used alone or in combination of two or more, but the present invention is not limited thereto. Preferably, a calcium phosphate such as α-tricalcium phosphate, β-tricalcium phosphate, dicalcium phosphate dibasic, tetracalcium phosphate, etc. may be used. More preferably, β-tricalcium phosphate (β-TCP) may be used.

In the present invention, the plaster may serve to increase the strength of the inorganic material for bone replacement, and is also a soluble material that may be dissolved in a body fluid. In the present invention, the plaster is characterized in that a volume of the plaster expands as calcium sulfate hemihydrate (CaSO₄·1/2H₂O; hereinafter referred to as CSH) in a particle state is filled into pores of the porous inorganic material and then combined with moisture to be converted into calcium sulfate dihydrate (CaSO₄·2H₂O; hereinafter referred to as CSD).

The plaster is combined with two molecules of water (H₂O) so that the plaster is present in a hydrated crystal state of CaSO₄·2H₂O (CSD). However, when the plaster is heated and dried under reduced pressure, the plaster may be processed into CaSO₄·1/2H₂O (CSH) or CaSO₄·H₂O (calcium sulfate monohydrate, CSM), both of which have a good binding force with water. Then, when the CSH thus prepared is penetrated into pores of the porous inorganic material (β-TCP, etc.) and then wet-treated to be reduced into CSD, a volume of the CSD expands in the pores of the porous inorganic material so that CSD is present in a state in which the pores are clogged with CSD without escaping from the pores due to physical impact. Therefore, a density of the porous inorganic material increases, and thus the porous inorganic material has an excellent compressive strength.

In the present invention, NaCl has an excellent biodegradation rate, and may enhance new bone formation capability in the pores since a concentration of NaCl around crystals temporally increases as NaCl is dissolved in the body so that a difference in concentration from the body fluid thus formed induces diffusion of a body fluid like osmosis.

In the high-strength synthetic bone for bone replacement according to the present invention, NaCl and plaster whose volume expands as calcium sulfate hemihydrate (CSH) in a particle state is filled into pores of the porous inorganic material and then combined with moisture to be converted into calcium sulfate dihydrate (CSD) may be mixed at a certain ratio at which the merits of both materials may be strengthened. However, a mixing ratio of NaCl and the plaster is preferably in a range of 1:4 to 1:99(based on weight). When the mixing ratio is converted into % by weight, the plaster may be used at 80 to 99% by weight, and NaCl may be used at 1 to 20% by weight, based on the total weight of the mixture of NaCl and the plaster.

In the present invention, each of the plaster and NaCl is preferably filled in a particle state having a diameter of 100 µm or less so that each of the plaster and NaCl penetrates into the pores of the porous inorganic material.

The high-strength synthetic bone for bone replacement according to the present invention may further include a polysaccharide filled into the pores of the porous inorganic material. The polysaccharide may induce the phase equilibrium in the body.

Also, the present invention provides a method for manufacturing a high-strength synthetic bone for bone replacement. Specifically, the method for manufacturing a high-strength synthetic bone for bone replacement according to the present invention may include mixing plaster and NaCl with a porous inorganic material to fill the plaster and NaCl into pores of the porous inorganic material, wherein a volume of the plaster expands as calcium sulfate hemihydrate (CSH) in a particle state is filled into the pores of the porous inorganic material and then combined with moisture to be converted into calcium sulfate dihydrate (CSD); wet-treating the porous inorganic material filled with the plaster and NaCl; and drying the wet-treated porous inorganic material.

Before the mixing, the porous inorganic material and the plaster are preferably sufficiently dried to remove moisture, and then mixed. For example, the porous inorganic material and the plaster may be dried at 20 to 50°C for 10 to 40 hours in a vacuum oven. The porous inorganic material and the plaster are ground when thoroughly dried. In this case, the porous inorganic material and the plaster having a diameter of 100 µm or less screened with a sieve are preferably used. The mixing of the respective materials may be performed using a powder mixer. A mixing time may, for example, be in a range of 1 to 60 minutes. The wet treatment may be performed using a method of injecting water, etc. A quantity of the injected water may vary according to the size or weight of the porous inorganic material, and may, for example, be in a range of 0.01 to 100 mL. The final drying may, for example, be performed at 20 to 50°C for 10 to 40 hours in a vacuum oven.

Hereinafter, the present invention will be described in further detail.

According to a preferred embodiment of the present invention, there is provided a high-strength synthetic bone for bone replacement in which a filler (a soluble material) penetrates into an inorganic material for bone replacement, characterized in that the inorganic material for bone replacement is β-tricalcium phosphate (β-TCP), and the filler includes CSH and NaCl.

In the present invention, first of all, each of the materials to be used for the inorganic material for bone replacement is selected as an absorbent material. Specifically, among the inorganic materials currently used, β-TCP, which is an absorbent material and may be considered to be the most actively studied, was selected as a research target. Also, CSH and NaCl which has higher solubility in the body than β-TCP and may increase a compressive strength are selected as the filler.

Since β-TCP has a chemical composition similar to a natural bone, and exhibits excellent biocompatibility with biological tissues, β-TCP has received much attention and has been studied as a material for synthetic bone implants. Since it is known that, when β-TCP is installed, bone-like apatite is generated at the interface between the material and bone tissue, β-TCP is directly or indirectly connected with the bone tissue.

A dissolution rate of β-TCP is highly affected by the chemical structure, crystallinity, and porosity of the material, pH of a solution, etc. β-TCP has been used as a material for bone regeneration since β-TCP is osteoconductive, and provides a suitable physical propensity to deposit new bone.

Since the inorganic materials for bone replacement are not osteoinductive materials but osteoconductive materials, the inorganic materials are preferably used as the porous materials having connected pores having a suitable size such that bone tissue can enter to grow in the materials, and have a desired property such that the inorganic materials have a biodegradation rate similar to the growth rate of new bone. However, the porous material has a reduced strength as a quantity of the pores increases. Since the strength of β-TCP is degraded when β-TCP is manufactured using a porous material, β-TCP has a problem in that moldability of the porous material such as block-type β-TCP is not easily maintained during surgery. When such a porous material is used as a bone graft material, a force used to firmly support an implant is lowered, and thus the implant may become loose due to the lack of alveolar bone during implant implantation. Owing to these problems, there is an urgent demand for materials capable of increasing the strength of a β-TCP block in the living body

In recent years, bone substitutes have been applied to fields of orthopedics (artificial hip joint, tarsal joints, etc.), fields of plastic surgery (fibula construction, maxillofacial bone reconstruction, etc.), fields of dental surgery (alveolar bone regeneration, alveolar bone construction, implant implantation, etc.). Therefore, when an efficient high-strength synthetic bone for bone replacement capable of reconstructing a defective bone is developed, products having technical superiority all over the world may be produced. When the high-strength synthetic bone for bone replacement developed according to the present invention is mass-produced, the high-strength synthetic bone for bone replacement is expected to create higher value-added business profits and have a high import substitution effect.

The present invention relates to improvement of compressive strength of the inorganic material used in the synthetic bone for bone replacement, preferably provides a high-strength synthetic bone for bone replacement in which calcium sulfate hemihydrate (CSH) and NaCl in a particle state penetrate into pores of β-tricalcium phosphate (β-TCP) and then are wet-treated such that the CSH penetrated into the pores is combined with moisture to form hydrated crystals of calcium sulfate dihydrate (CSD) in order to expand a volume of the CSH in the pores, thereby preventing the escape of the filler due to a physical force.

According to the present invention, the high-strength synthetic bone for bone replacement may be manufactured since the filler (CSH, NaCl, etc.) penetrated into the pores may serve to increase the compressive strength of the porous inorganic material (a β-TCP block, etc.) and also improve new bone conduction capability due to the pores reduced during a process in which the filler is eluted into a body fluid in the body. Specifically, the CSH is penetrated into the pores of the porous inorganic material so that a 1/2H₂O hydrated product is converted into a 2H₂O hydrated crystal of CSD to expand a volume of the CSH in the pores. As a result, when the filler is filled into the pores once, the escape of the filler from the pores may be prevented due to a physical force, resulting in increased strength of the inorganic material. As a filler which penetrates together with the CSH, NaCl is also dissolved in the body fluid present in the pores of the inorganic material after implantation surgery to increase a concentration of NaCl in the pores, and such a partial difference in concentration in the pores induces a phase equilibrium reaction in which the body fluid is rapidly attracted into the pores like osmosis, thereby improving blood circulation so as to aid in forming a new bone.

Therefore, the high-strength synthetic bone for bone replacement according to the present invention may have an increased ease in molding a pre-surgical material during surgery for implantation of a bone substitute and may maintain a shape of a molded product in an original state in the body for a predetermined period of time after the surgery since the high-strength synthetic bone for bone replacement has an increased compressive strength. At the same time, the high-strength synthetic bone for bone replacement according to the present invention is expected to be widely used as a better material capable of replacing conventional synthetic bones for bone replacement since a change in concentration of a body fluid in pores of an inorganic material with the dissolution of NaCl as a filler induces a phase equilibrium reaction similar to osmosis to improve blood circulation in the pores.

Hereinafter, the present invention will be described in further detail with reference to examples thereof. However, it should be understood that the following examples are just preferred examples for the purpose of illustration only and is not intended to limit or define the scope of the invention.

### [Example 1]

Materials as listed in Table 1 were used to manufacture a high-strength synthetic bone for bone replacement into which a mixed filler including 1% NaCl and CSH was penetrated.

**[Table 1]**

| Materials used in this experiment | |
|---|---|
| Materials | Manufacturer |
| cylindrical β-Tricalcium phosphate | Ossgen |
| CSH | SIGMA-ALDRICH |
| NaCl | SIGMA-ALDRICH |

### A. Preparation of cylindrical β-TCP block

A commercially available cylindrical β-TCP block having a diameter of 5 mm and a length of 10 mm was dried at 37°C for 24 hours under reduced pressure in a vacuum oven (Jeio Tech Co. Ltd., OV-12) to prepare a cylindrical β-TCP block from which moisture was removed.

### B. Preparation of mixed filler

Each of CSH and NaCl in a powder state was weighed and prepared as listed in Table 2 (units: % by weight), and mixed using a powder mixer (KM Tech, LS-300). Then, the resulting mixture was ground using a mortar. The mixed filler including each of the ground powders was filtered through a 100 µm sieve, and the mixed filler passing through the 100 µm sieve was dried at 37°C for 24 hours under reduced pressure in a vacuum oven to prepare a mixed filler from which moisture was removed.

**[Table 2]**

| Mixing ratio of fillers (CSH and NaCl) (based on weight) | | | |
|---|---|---|---|
| Sample No. | mixed filler | CSH | NaCl |
| 1 | 1% NaCl-CSH | 99 | 1 |
| 2 | 5% NaCl-CSH | 95 | 5 |
| 3 | 10% NaCl-CSH | 90 | 10 |
| 4 | 15% NaCl-CSH | 85 | 15 |
| 5 | 20% NaCl-CSH | 80 | 20 |

### C. Preparation of high-strength synthetic bone for bone replacement into which 1% NaCl-CSH mixed filler is penetrated

The mixed filler 1 prepared during a process of preparing the mixed filler was added to a sieve shaker (Chunggye, CG-212S), and the cylindrical β-TCP block prepared during a process of preparing the cylindrical β-TCP block was shaken for 20 minutes in the sieve shaker containing the mixed filler 1. When the shaking was completed, a surface of the cylindrical β-TCP block was cleaned with a brush, and the β-TCP block was allowed to absorb 2ml of water, and then dried at 37°C for 24 hours under reduced pressure in a vacuum oven.

### [Example 2]

A high-strength synthetic bone for bone replacement into which a 5% NaCl-CSH mixed filler was penetrated was manufactured in the same manner as in Example 1, except that a mixed filler 2 was used instead of the mixed filler 1 in C of Example 1.

### [Example 3]

A high-strength synthetic bone for bone replacement into which a 10% NaCl-CSH mixed filler was penetrated was manufactured in the same manner as in Example 1, except that a mixed filler 3 was used instead of the mixed filler 1 in C of Example 1.

### [Example 4]

A high-strength synthetic bone for bone replacement into which a 15% NaCl-CSH mixed filler was penetrated was manufactured in the same manner as in Example 1, except that a mixed filler 4 was used instead of the mixed filler 1 in C of Example 1.

### [Example 5]

A high-strength synthetic bone for bone replacement into which a 20% NaCl-CSH mixed filler was penetrated was manufactured in the same manner as in Example 1, except that a mixed filler 5 was used instead of the mixed filler 1 in C of Example 1.

### [Example 6]

Materials listed in Table 3 were used to prepare a simulated body fluid (SBF) having an ion concentration similar to human plasma. The materials listed in Table 3 below were sequentially dissolved in 700 mL of double distilled water, and then buffered with (CH₂OH₃)CNH₂ and 1 M hydrochloric acid at pH 7.25 and 37°C to prepare an SBF.

**[Table 3]**

| Reagents for simulated body fluid | |
|---|---|
| Solution | Volume |
| NaCl | 7.996 g |
| NaHCO₃ | 0.35 g |
| KCl | 0.224 g |
| K₂HPO₄·3H₂O | 0.228 g |
| MgCl₂·6H₂O | 0.305 g |
| 1M HCl | 40 mL |
| CaCl₂ | 0.278 g |
| Na₂SO₄ | 0.071 g |
| (CH₂OH₃)CNH₂ | 6.057 g |

To check whether each of the NaCl-CSH mixed fillers prepared in Examples 1, 2, 3, 4, and 5 penetrating the high-strength synthetic bone for bone replacement was smoothly dissolved and eluted in the body and reduced to an original state of the porous inorganic material, each of NaCl-CSH mixed fillers was deposited in the simulated body fluid prepared in Example 6, which had an ion concentration similar to human plasma, for different times, and then dried at 37°C for 24 hours under reduced pressure in a vacuum oven.

### [Example 7]

A cylindrical β-TCP block into which the 1% NaCl-CSH mixed filler was penetrated was deposited in the simulated body fluid for 1 week, taken out, and then dried at 37°C for 24 hours under reduced pressure in a vacuum oven.

### [Example 8]

A cylindrical β-TCP block into which the 1% NaCl-CSH mixed filler was penetrated was deposited in the simulated body fluid for 2 weeks, taken out, and then dried at 37°C for 24 hours under reduced pressure in a vacuum oven.

### [Example 9]

A cylindrical β-TCP block into which the 5% NaCl-CSH mixed filler was penetrated was deposited in the simulated body fluid for 1 week, taken out, and then dried at 37°C for 24 hours under reduced pressure in a vacuum oven.

### [Example 10]

A cylindrical β-TCP block into which the 5% NaCl-CSH mixed filler was penetrated was deposited in the simulated body fluid for 2 weeks, taken out, and then dried at 37°C for 24 hours under reduced pressure in a vacuum oven.

### [Example 11]

A cylindrical β-TCP block into which the 10% NaCl-CSH mixed filler was penetrated was deposited in the simulated body fluid for 1 week, taken out, and then dried at 37°C for 24 hours under reduced pressure in a vacuum oven.

### [Example 12]

A cylindrical β-TCP block into which the 10% NaCl-CSH mixed filler was penetrated was deposited in the simulated body fluid for 2 weeks, taken out, and then dried at 37°C for 24 hours under reduced pressure in a vacuum oven.

### [Example 13]

A cylindrical β-TCP block into which the 15% NaCl-CSH mixed filler was penetrated was deposited in the simulated body fluid for 1 week, taken out, and then dried at 37°C for 24 hours under reduced pressure in a vacuum oven.

### [Example 14]

A cylindrical β-TCP block into which the 15% NaCl-CSH mixed filler was penetrated was deposited in the simulated body fluid for 2 weeks, taken out, and then dried at 37°C for 24 hours under reduced pressure in a vacuum oven.

### [Example 15]

A cylindrical β-TCP block into which the 20% NaCl-CSH mixed filler was penetrated was deposited in the simulated body fluid for 1 week, taken out, and then dried at 37°C for 24 hours under reduced pressure in a vacuum oven.

### [Example 16]

A cylindrical β-TCP block into which the 20% NaCl-CSH mixed filler was penetrated was deposited in the simulated body fluid for 2 weeks, taken out, and then dried at 37°C for 24 hours under reduced pressure in a vacuum oven.

### [Example 17]

*In vitro* studies were performed on the examples, as follows.

### (1) Compressive strength

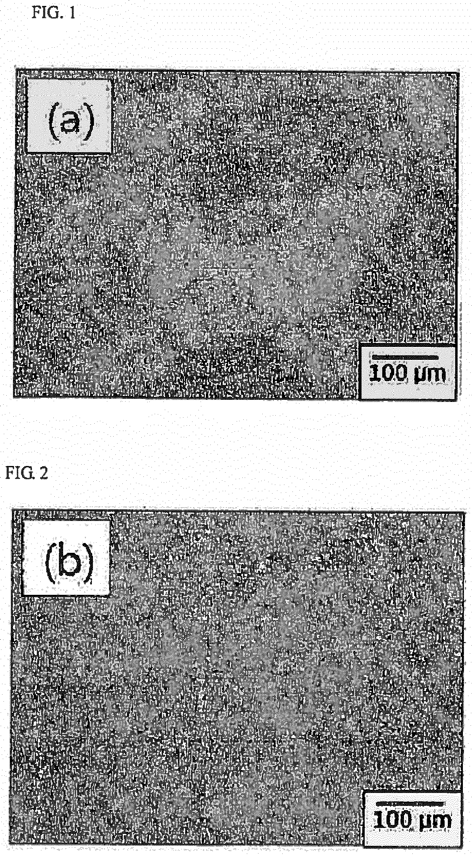

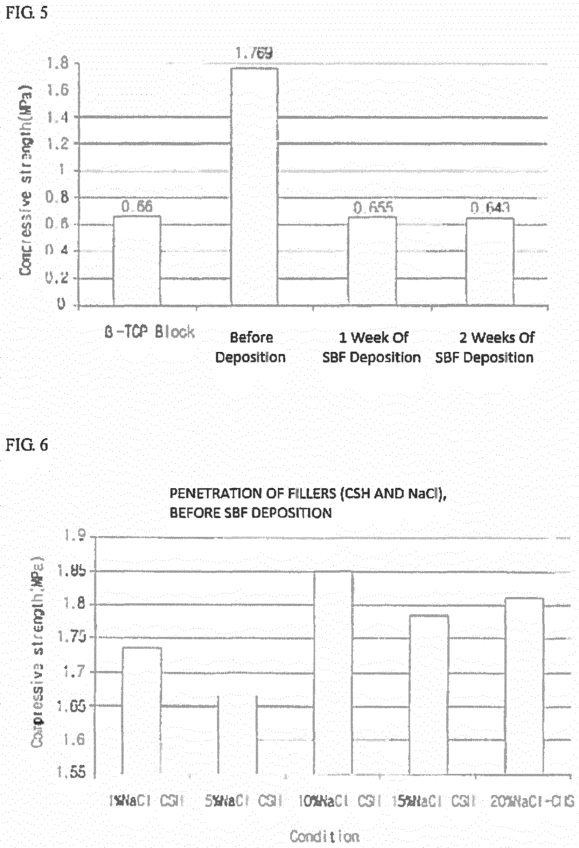

The compressive strength of cylindrical β-TCP having a height of 10 mm and a diameter of 5 mm was measured at a crosshead speed of 1 mm/min using a universal testing machine (Instron 3366, U.S.A.). FIGS. 2 to 5 are graphs illustrating changes in compressive strength according to the mixing ratios and conditions for SBF deposition in the high-strength synthetic bone for bone replacement according to the present invention. As shown in FIGS. 2 to 5, it can be seen that the compressive strength increased as the fillers, CSH and NaCl, were filled into the porous inorganic material, and the compressive strength was reduced to a level similar to an original state as the CSH and NaCl were deposited and then dissolved in SBF.

### (2) Surface observation

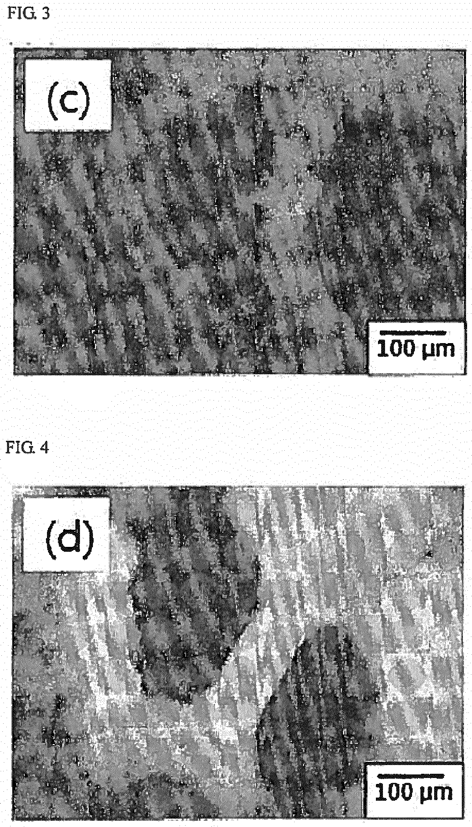

The high-strength synthetic bone for bone replacement thus manufactured was deposited and dissolved in SBF, and then dried. Thereafter, a surface of the high-strength synthetic bone for bone replacement was observed under a USB microscope (Ecwox, K89) with a 1,000× magnification to check a change in surface shape. FIGS. 1 to FIG. 4 are microscope images showing surface morphologies of the inorganic materials according to the present invention: FIG. 1A is an image of cylindrical β-TCP at the beginning, FIG. 2B is an image of cylindrical β-TCP after plaster is mixed with NaCl, FIG. 3C is an image of cylindrical β-TCP after 1 week of impregnation into SBF, and FIG. 4D is an image of cylindrical β-TCP after 2 weeks of impregnation into SBF. As shown in FIGS. 1 to FIG. 4, it can be seen that the quantity and size of the pores of the porous inorganic material were reduced as the plaster and NaCl were penetrated into the pores, and the quantity and size of the pores increased to levels similar to the original state as the plaster and NaCl were deposited and then dissolved in SBF.

In conclusion, in the present invention, the following results are obtained when the plaster and NaCl are penetrated into the pores of β-TCP that is a cylindrical inorganic material for bone replacement.
1. The quantity and size of the pores of the cylindrical β-TCP decreased when the plaster and NaCl are penetrated into the pores.
2. The compressive strength increases when the plaster and NaCl are penetrated into the pores of the cylindrical β-TCP.
3. The plaster and NaCl were dissolved and eluted when the high-strength synthetic bone for bone replacement was deposited in a simulated body fluid (SBF) for 1 week and 2 weeks. As a result, the quantity and size of the pores increase to levels similar to the original state, and the compressive strength decreases to a level similar to the original state.

## Claims

1. A synthetic bone for bone replacement, comprising:
a porous inorganic material;
plaster whose volume expands as calcium sulfate hemihydrate (CSH) in a particle state is filled into pores of the porous inorganic material and then combined with moisture to be converted into calcium sulfate dihydrate (CSD); and
NaCl filled into the pores of the porous inorganic material.

2. The synthetic bone for bone replacement of claim 1, wherein the porous inorganic material is a mixture of one or two or more selected from the group consisting of β-tricalcium phosphate, α-tricalcium phosphate, dicalcium phosphate dibasic, tetracalcium phosphate, hydroxyapatite, calcium phosphate cement, calcium carbonate, calcium sulfate, a bioactive glass ceramic, and silica.

3. The synthetic bone for bone replacement of claim 1, further comprising a polysaccharide filled into the pores of the porous inorganic material.

4. The synthetic bone for bone replacement of claim 1, wherein each of the plaster and NaCl is filled in a particle state having a diameter of 100 µm or less.

5. The synthetic bone for bone replacement of claim 1, wherein a mixing ratio of the NaCl and plaster is in a range of 1:4 to 1:99 (based on weight).

6. A method for manufacturing a synthetic bone for bone replacement, comprising:
mixing plaster and NaCl with a porous inorganic material to fill the plaster and NaCl into pores of the porous inorganic material, wherein a volume of the plaster expands as calcium sulfate hemihydrate (CSH) in a particle state is filled into the pores of the porous inorganic material and then combined with moisture to be converted into calcium sulfate dihydrate (CSD);
wet-treating the porous inorganic material filled with the plaster and NaCl; and
drying the wet-treated porous inorganic material.
